Europäisches Patentamt

European Patent Office

Office européen des brevets

(19)

(11) **EP 1 019 108 B1**

(12) **FASCICULE DE BREVET EUROPEEN**

(45) Date de publication et mention
de la délivrance du brevet:
**02.05.2002  Bulletin 2002/18**

(51) Int Cl.7: **A61L 27/00**

(21) Numéro de dépôt: **97900225.0**

(22) Date de dépôt: **02.01.1997**

(86) Numéro de dépôt international:
**PCT/FR97/00007**

(87) Numéro de publication internationale:
**WO 97/26024 (24.07.1997 Gazette 1997/32)**

(54) **COMPOSITE MACROPOREUX SUPPORT DE SUBSTANCE(S) MEDICAMENTEUSE(S) UTILISABLE COMME MATERIAU DE RECONSTITUTION OSSEUSE ET PROCEDE D'ELABORATION**

MAKROPORÖSES KOMPOSIT ALS TRÄGERSUBSTANZ FÜR MEDIKAMENTE UND ZUR VERWENDUNG ALS KNOCHENERSATZMATERIAL

MACROPOROUS COMPOSITE FOR CARRYING ONE OR MORE MEDICINAL SUBSTANCES AND FOR USE AS A BONE RECONSTRUCTION MATERIAL, AND METHOD FOR MAKING SAME

(84) Etats contractants désignés:
**AT BE CH DE DK ES FI GB GR IE IT LI LU MC NL PT SE**

(30) Priorité: **15.01.1996  FR 9600560**

(43) Date de publication de la demande:
**19.07.2000   Bulletin 2000/29**

(73) Titulaire: **UNIVERSITE DE RENNES I**
**F-35000 Rennes (FR)**

(72) Inventeurs:
• **LUCAS, Anita**
  **F-35000 Rennes (FR)**
• **MICHEL, Jean-François**
  **F-35700 Rennes (FR)**

• **GAUDE, Jean**
  **F-35690 Acigné (FR)**
• **CAREL, Claude**
  **F-35000 Rennes (FR)**

(74) Mandataire: **Vidon, Patrice**
  **Cabinet Patrice Vidon**
  **Le Nobel (Bât. A)**
  **Technopôle Atalante**
  **2, allée Antoine Becquerel**
  **BP 90333**
  **35703 Rennes Cedex 7 (FR)**

(56) Documents cités:
**EP-A- 0 022 724       EP-A- 0 159 087**
**EP-A- 0 395 187       WO-A-87/07826**
**WO-A-94/26322        US-A- 3 890 107**

EP 1 019 108 B1

## Description

L'invention concerne le domaine des biomatériaux pouvant être utilisés pour remplacer une partie du système osseux.

Les biomatériaux sont des matériels utilisés pour remplacer une partie du système vivant ou pour fonctionner avec lui. Ils sont constitués par des matériaux étrangers au receveur, implantés chez celui-ci pour restaurer la morphologie et/ou la fonction de tissus altérés par des traumatismes ou une maladie. Ils présentent donc tous l'originalité de devoir travailler sous contrainte biologique.

Le biomatériau doit satisfaire à des caractéristiques physico-chimiques appropriées au site où il sera implanté et à la fonction qu'il devra remplir. Les chances de succès d'un biomatériau dans l'organisme résultent du produit de multiples facteurs qui devront tous être maîtrisés.

Du point de vue des implants réalisés avec de tels biomatériaux, la biocompatibilité de ceux-ci se définit comme le contrôle des réactions de l'organisme à leur égard pendant toute le durée de contact entre le biomatériau en question et cet organisme. Propriétés mécaniques et biologiques d'un tel produit sont donc étroitement liées.

L'invention se rapporte plus précisément aux biomatériaux utilisables en tant que matériaux de reconstitution osseuse.

Dans ce domaine, le corail a été proposé et utilisé de façon importante depuis une dizaine d'années. Ce matériau naturel possède un exosquelette minéral à porosité ouverte, formé majoritairement d'aragonite. L'aragonite est une variété allotropique du carbonate de calcium. qui cristallise dans un système orthorhombique avec les paramètres de maille a =4 ,9623(3) Å, b = 7,968(1) Å, c = 5,7439 Å pour Z = 4. Sa densité théorique est d = 2,93.

Il a été proposé dans l'état de la technique d'utiliser des préparations à base de corail comme matériaux de reconstitution osseuse dans le domaine de la parodontologie (maladies parodontales, chirurgie osseuse correctrice des pertes de substance). Ce matériau est utilisé dans ce cadre sous forme de granules de taille allant de 300 à 450 µm pour le comblement des défauts parodontaux et de 600 à 1000 µm pour le comblement d'alvéoles après extraction dentaire. Les granules sont préférés aux blocs conduisant à l'exfoliation du fragment corallien après son implantation, la taille des particules recommandée pour le comblement des défauts parodontaux étant alors de 300 à 500 µm.

La demande de brevet français FR 2637502 décrit ainsi un matériau de reconstitution osseuse constitué de corail madréporaire lavé de toute substance organique d'origine et renfermant un accélérateur organique d'ostéogenèse se présentant sous la forme d'un gel protéique à base de collagène de type I.

La demande de brevet internationale WO 94/26322 divulgue quant à elle un matériau poreux constitué d'un squelette de corail (de genre Porites, Acropora, Goniopora, Lobophyllia, Simphyllia ou Millipora) et d'un facteur de croissance pouvant être ostéoinducteur.

La demande de brevet européen EP 398187 décrit quant à elle un biomatériau composé de corail recouvert d'une couche d'hydroxyapatite.

L'expérience a montré que le corail possédait une biocompatibilité élevée confirmant son intérêt en tant que matériau de reconstitution osseuse.

Toutefois, un tel matériau présente l'inconvénient majeur d'induire un coût de revient fort élevé suscité par son traitement en vue de son utilisation comme matériau implantable. En pratique le corail récolté dans la nature doit être fractionné puis lavé et incubé pendant plusieurs jours dans des produits tels que l'hypochlorite de soude permettant de le débarrasser de tout substance organique. Une telle incubation doit être effectuée de façon à permettre au produit utilisé de pénétrer dans l'ensemble de la structure poreuse du corail. Les blocs de corail ainsi traités doivent ensuite être soit concassés et sélectionnés par gammes de dimension des granules, soit usinés pour être adaptés géométriquement aux sites d'implantation, puis être de nouveau lavés.

De plus, le corail présente un porosité qu'il n'est pas possible de contrôler. En fonction de la vitesse de résorption recherchée, il convient alors de choisir tel ou tel type de corail en fonction de sa porosité naturelle.

L'objectif principal de la présente invention est de proposer un biomatériau utilisable en tant que matériau de reconstitution osseuse permettant de résoudre ce problème.

Un autre objectif de l'invention est aussi de divulguer un tel matériau pouvant être réalisé de façon à présenter des propriétés, notamment de porosité, modulables en fonction de l'utilisation qu'il est envisagé d'en faire.

Encore un autre objectif de l'invention est de proposer un tel matériau pouvant inclure une ou plusieurs substances médicamenteuses, en vue d'une administration avec relargage différé de ces substances.

Ces différents objectifs, ainsi que d'autres qui apparaitront par la suite sont atteints, grâce à l'invention qui concerne un composite macroporeux, utilisable en tant que matériau de reconstitution osseuse, caractérisé en ce qu'il est constitué par l'association d'aragonite synthétique et d'au moins une substance médicamenteuse.

Un tel composite présente de nombreux avantages.

En premier lieu, s'agissant d'un matériau à base d'aragonite, il peut se résorber lentement *in situ*. La substance médicamenteuse qu'il inclut pourra ainsi être relarguée progressivement dans l'organisme et avoir un effet retard.

Le fait d'inclure dans le substrat aragonitique synthétique au moins une substance médicamenteuse offre de plus des potentialités thérapeutiques intéressantes par action locale de cette substance médicamenteuse à haute concentration.

Par ailleurs et surtout, l'aragonite utilisée étant synthétique, il sera possible de moduler certains paramètres lors de sa synthèse. On pourra ainsi obtenir un substrat aragonitique présentant des pores de taille plus ou moins importante, une porosité ouverte ou fermée, ou encore un substrat permettant la réalisation d'un matériau plus ou moins dense ou plus ou moins rigide. On pourra de plus moduler la concentration nominale de la ou des substances médicamenteuses dans le composite. L'utilisation d'aragonite synthétique présente ainsi un avantage considérable par rapport aux substrats naturels dont on ne peut évidemment pas faire varier la taille et la nature des pores en fonction de l'objectif recherché et dans lesquels l'introduction de substances étrangères en quantité déterminée n'es pas réalisable simplement.

Enfin, on notera que le prix de revient d'un tel composite sera bien inférieur à ceux des préparations de l'état de la technique à base de corail. Plus précisément, on estime que ce prix de revient serait environ 10 fois inférieur à celui des préparations à base de corail.

Le composite selon l'invention pourra notamment être utilisé en odontologie, en particulier en chirurgie buccale et en parodontologie, et en orthopédie chirurgicale. Toutefois, on notera que le domaine d'application de tels composites n'est pas limité au seul domaine dentaire et que l'extension ultérieure à la chirurgie osseuse en générale est tout à fait envisageable.

Préférentiellement, l'aragonite synthétique représentera entre environ 85 % et 99,5 % de la masse totale dudit composite. Cependant, en fonction de l'utilisation qui sera faite du composite réalisé, on pourra envisager, dans certains cas de sortir d'une telle fourchette.

Selon une variante particulièrement intéressante, ladite substance médicamenteuse est un antibiotique. On connaît l'intérêt des antibiotiques et, comme mentionné, ci-dessus, le composite selon l'invention permet l'administration avec effet retard de la substance médicamenteuse incluse dans le composite. Ceci est particulièrement intéressant pour les antibiotiques. Par ailleurs, on estime qu'un tel antibiotique ainsi associé à un substrat aragonitique pourra avoir une action locale à haute concentration pour une quantité 100 à 1000 fois moindre que celle nécessitée par une administration par voie générale.

Avantageusement ledit antibiotique est un antibiotique à large spectre tel que du métronidazole, dont l'action sur les germes anaérobies est recherchée dans la chirurgie osseuse.

L'invention concerne également un procédé de fabrication d'un tel composite macroporeux caractérisé en ce qu'il consiste à réaliser un mélange comprenant des grains d'aragonite synthétique et au moins un agent porogène, à compacter ledit mélange et à chauffer le produit obtenu de façon à éliminer ledit agent porogène, au moins une substance médicamenteuse étant incluse avant ou après ladite étape d'élimination dudit agent porogène.

La ou les substance(s) médicamenteuse(s) pourra(ont) être ajoutée(s), s'il s'agit d'une ou de poudre(s) avant le chauffage, s'il s'agit d'une ou de substance(s) fluide(s) après le chauffage, par imbibition ou aspiration.

Préférentiellement, ledit agent porogène est un composé solide se présentant aussi sous forme de poudre. La taille des grains de la poudre constituant ledit agent porogène correspondra sensiblement à la taille des pores du composite. Selon une variante intéressante, la taille de ces grains sera préférentiellement comprise entre 100 et 1000 μm.

Egalement préférentiellement, ledit agent porogène est du naphtalène ($C_{10}H_8$). Comme il sera expliqué plus en détail ci-après, il a été observé que la présence de naphtalène lors du compactage confère un caractère multidirectionnel à l'orientation des grains d'aragonite permettant d'éviter la fracture transversale des comprimés obtenus grâce au procédé selon l'invention. On pourra toutefois également envisager d'utiliser d'autres types d'agents porogènes connus de l'état de l'art.

Dans le cas préférentiel où l'agent porogène utilisé est du naphtalène, ladite étape de chauffage mise en oeuvre dans le cadre du procédé selon l'invention est avantageusement effectuée à une température d'environ 110°C conduisant à la sublimation du naphtalène. On comprendra que, dans le cas où l'on choisira un autre agent porogène que le naphtalène, on pourra utiliser une autre température de chauffage.

D'une manière générale, lorsque ladite substance médicamenteuse est introduite avant ladite étape d'élimination dudit agent porogène ladite étape de chauffage sera avantageusement effectuée à des températures de l'ordre de 110°C.

Toutefois, lorsque, selon une variante de l'invention, ladite substance médicamenteuse est introduite après ladite étape d'élimination dudit agent porogène ladite étape de chauffage pourra être effectuée à des températures de l'ordre de 400°C ou plus.

Egalement préférentiellement, ladite étape de compactage est effectuée par pressage uniaxial. Un tel pressage présente l'avantage de pouvoir être facilement mis en oeuvre. On aurait pu craindre que ce type de pressage conduise à une orientation préférentielle des cristaux d'aragonite. Or, il s'avère que la présence dans le mélange utilisé de grains de naphtalène et la sublimation de cet agent porogène conduisent à l'obtention avantageuse de cristaux relativement désorientés.

Il pourra être envisager de réaliser le substrat aragonitique selon plusieurs voies de synthèse connues de l'état de la technique. L'aragonite, dans les conditions ambiantes de température et de pression, n'est pas la variété thermodynamiquement stable du carbonate de calcium, ce qui rend son obtention plus difficile que celle de la calcite qui est la variété stable.

Une des voies d'accès possible est de reproduire en laboratoire les conditions de précipitation en phase aqueuse présentes dans la nature, sous des températures et des pressions ordinaires. L'obtention de l'aragonite peut ainsi être obtenue à partir d'eau de mer, naturelle ou artificielle, concentrée en ions $Ca^{2+}$. La précipitation peut alors intervenir après génération d'ions carbonate, à partir de solution saturées en hydrogénocarbonate, par enlèvement de $CO_2$.

Une autre voie consiste à ajouter les ions carbonate à la solution d'ions $Ca^{2+}$ sous la forme d'un carbonate soluble comme $Na_2CO_3$. Dans ce cas, l'addition d'ions favorisant la précipitation de l'aragonite est indispensable.

L'inconvénient majeur de ces méthodes est une cinétique lente.

La carbonatation de l'eau de chaux contenant parfois des additifs, ou d'une solution de nitrate de calcium, en milieu basique permet également, dans certaines conditions, d'obtenir l'aragonite.

D'autres procédés sont également connus mais conduisant à des quantités faibles d'aragonite et/ou en présence d'autres variétés (calcite, vatérite).

L'invention concerne donc également un procédé de synthèse d'aragonite synthétique permettant l'obtention d'aragonite contenant éventuellement une autre variété en tant qu'impureté, notamment la calcite, dans des conditions économiquement intéressantes. Un tel procédé est caractérisé en ce qu'il consiste à mettre en contact de l'hydrogénocarbonate de potassium avec du chlorure de calcium, à raison de 2 moles d'hydrogénocarbontate de potassium pour une mole de chlorure de calcium, en solution à l'ébullition et à pHcompris entre 7 et 9.

L'invention sera plus facilement comprise grâce à la description qui va suivre d'un mode de réalisation non limitatif de celle-ci en référence aux dessins, dans lesquels :

- la figure 1 représente le diagramme de diffraction des rayons X de l'aragonite obtenue par synthèse dans le cadre du présent exemple de réalisation ;
- la figure 2 représente une vue schématique d'un moule à pastiller utilisé pour le compactage d'un mélange aragonite-métronidazole-naphtalène ;
- la figure 3 représente l'évolution de la densité géométrique des composites obtenus, en fonction du pourcentage massique de naphtalène utilisé ;
- la figure 4 représente l'évolution de la porosité des composites obtenus, en fonction du pourcentage massique de naphtalène utilisé.

Synthèse de l'aragonite

Une solution de chlorure de calcium 0,1 M a été additionnée, en continu, à une solution d'hydrogénocarbonate de potassium 0,1 M présentant un pH de 8, 6 à 20° C, à raison de 2 moles d'hydrogénocarbonate de potassium pour 1 mole de chlorure de calcium. L'addition a été effectuée à chaud (entre 90° C et 100° C) à l'aide d'un dispositif d'addition contrôlée. La solution a été placée sur une platine chauffante à agitateur magnétique, l'agitation étant assurée par un barreau aimanté tournant à une vitesse de 500 tours/minute.

Cette addition a mené à une précipitation de carbonate de calcium dans sa variété aragonite, selon la réaction pouvant être symbolisée par :

$$2\ KHCO_3 + CaCl_2 \rightarrow CO_2 + H_2O + CaCO_3 + 2\ KCL$$

Le précipité obtenu a été filtré sur papier, à l'aide d'un Büchner préalablement porté à la température de séchage. Ensuite, le précipité a été lavé à l'eau désionisée bouillante ($\approx$ 100° C) puis séché dans une étuve portée à 110 $\pm$ 2° C.

Il a été vérifié par diffraction des rayons X qu'un tel procédé de synthèse permet d'obtenir le carbonate de calcium $CaCO_3$ sous forme d'aragonite en absence d'autres phases.

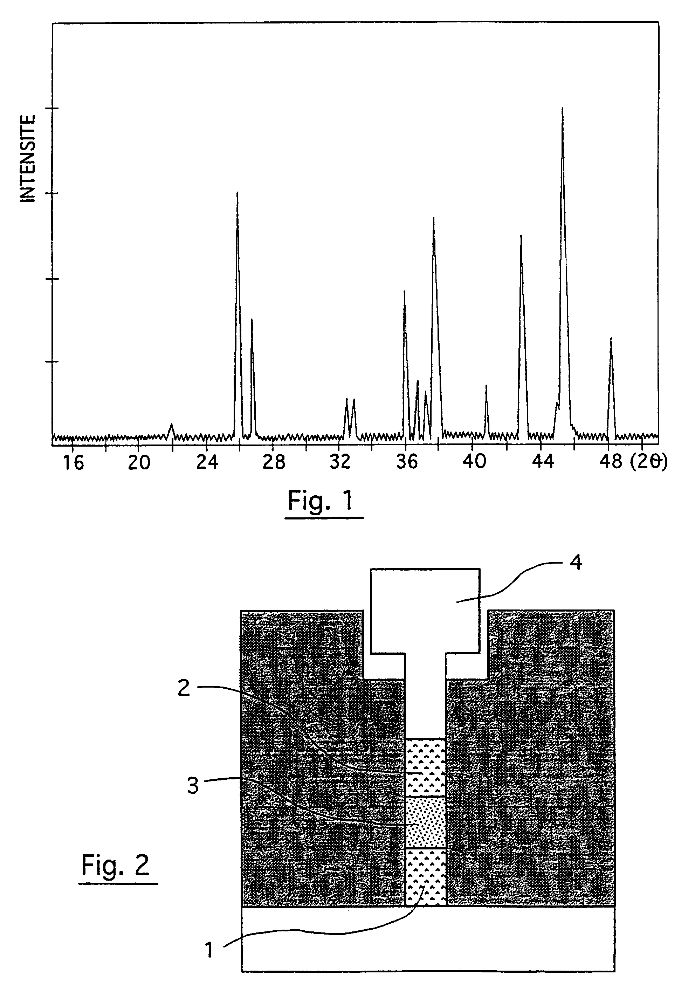

Plus précisément le diagramme de diffraction des rayons X a été enregistré à température et à pression ambiantes. Ce diagramme est représenté sur la figure 1.

L'analyse granulométrique en volume du produit obtenu a montré que tous les grains présentaient un diamètre équivalent inférieur à 62 $\mu$m. La distribution en nombre a indiqué que 99, 8 % des particules présentaient un diamètre équivalent inférieur à 1, 8 $\mu$m.

Préparation du composite

Du naphtalène présentant une pureté supérieure à 99 % (Scintillation grade de chez Aldrich) a été broyé manuellement dans un mortier et tamisé grâce à un tamiseur à vibrations verticales pendant 15 minutes. Les ouvertures des tamis du tamiseur ont successivement été de 250, 160 puis 100 μm.

Une fraction déterminée de naphtalène a été récupérée à partir du tamis correspondant à une ouverture de 100 μm. En faisant abstraction de la tendance naturelle des grains de naphtalène à s'agglomérer, il a pu être vérifié que le diamètre des grains récupérés se situait entre 100 et 160 μm.

Par ailleurs, du métronidazole (1-(2-hydroxy-éthyl)-2-méthyl-5-nitro-imidazole) se présentant sous la forme d'une poudre cristalline très légèrement jaune à 99,95 %, fourni par la Société Spécia (Groupe Rhône Poulenc Rorer Spécia), a été broyé à la main et tamisé grâce à un tamiseur à vibrations verticales pendant 15 minutes. Les ouvertures des tamis du tamiseur sont de 160 et 100 μm.

Le métronidazole ainsi tamisé a ensuite été mélangé manuellement sans broyage avec de la poudre d'aragonite obtenue selon le procédé décrit ci-dessus puis avec des grains de naphtalène broyés.

Trois mélanges différents ont ainsi été réalisés, dans lesquels, le métronidazole broyé représentaient toujours 5 % de la masse totale dudit mélange, le naphtalène représentant successivement 28,6 %, 21,1 % et 14,6 % de cette masse totale.

une masse de 0,1 g de chacun des mélanges d'aragonite synthétique, de métronidazole et de naphtalène réalisés a ensuite été placée entre les deux cylindres métalliques 1, 2 d'un moule à pastiller (SPECAC - marque déposée -, diamètre 6mm) tel que représentée de façon schématique sur la figure 2. Le compactage des mélanges 3 a été effectué par application d'une forte pression uniaxiale de 900 MPa (9 kbar) à l'aide d'un piston 4. Cette pression a été maintenue pendant 5 minutes, puis a été diminuée progressivement jusqu'à la pression atmosphérique.

L'élimination du naphtalène a été obtenue par chauffage des pastilles dans un four muni d'un régulateur électronique à 90 °C, sous vide primaire, pendant 6 heures. A la suite de ce traitement, les pastilles ont été placées à 110° C, sous pression atmosphérique pendant 16 à 17 heures, puis refroidies en atmosphère sèche dans un dessiccateur .

Une variante de préparation des pastilles consiste, après avoir débarrassé le mélange aragonite/porogène de celui-ci à des températures habituelles, à porter l'aragonite poreuse ainsi obtenue à des températures de l'ordre de 400° C ou plus, éventuellement sous dioxyde de carbone $CO_2$, pour obtenir un frittage permettant d'augmenter la solidité du comprimé. Celui-ci peut ensuite être chargé après refroidissement en substance(s) médicamenteuse(s) par imbibition ou aspiration.

Caractéristique des pastilles obtenues

Les produits obtenu à la suite de l'opération décrite ci-dessus se présentent sous la forme de pastilles exemptes de naphtalène montrant une épaisseur variant de 1,92 mm à 2,04 mm pour un diamètre de 6 mm.

Le tableau ci-après indique la densité géométrique et la porosité des trois pastilles correspondant aux mélanges ci-dessus.

| % (masse) naphtalène | Densité géométrique | Porosité (%) |
|---|---|---|
| 28,6 | 1,28 ± 0,02 | 54,0 ± 1,3 |
| 21,1 | 1,47 ± 0,03 | 47,1 ± 1,6 |
| 14,6 | 1,64 ± 0,03 | 41,0 ± 1,6 |

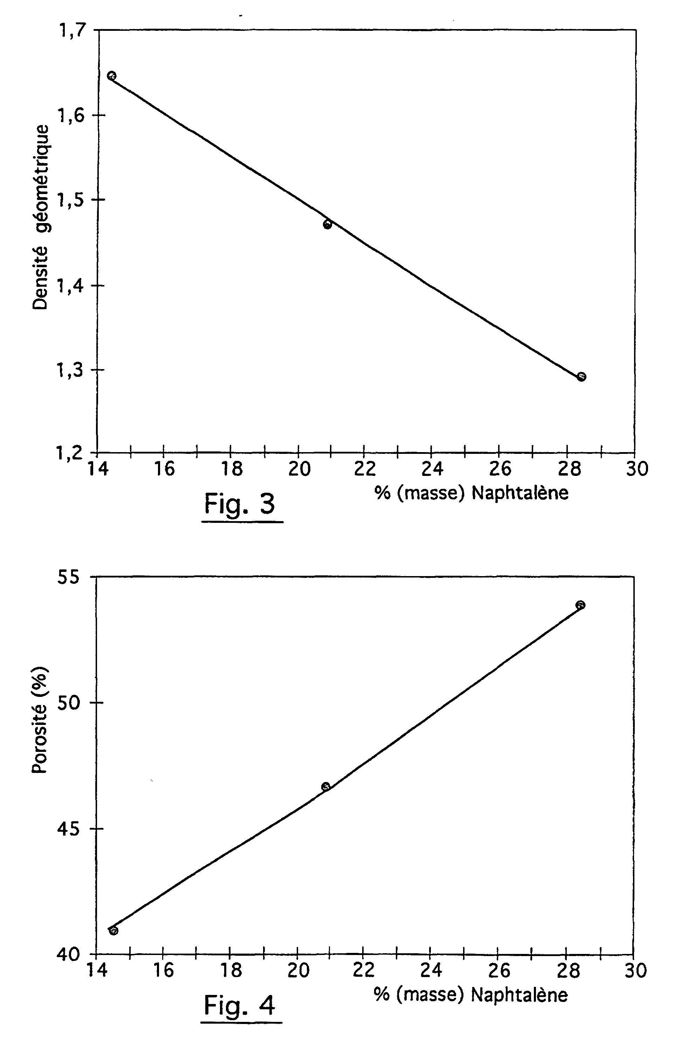

Comme on peut le voir sur les figures 3 et 4 reprenant les données du tableau ci-dessus, la densité géométrique du composite obtenu décroît sensiblement linéairement lorsque la teneur en agent porogène utilisée augmente et, inversement, la porosité de ce composite augmente sensiblement linéairement lorsque la teneur en agent porogène utilisée augmente.

L'observation des échantillons en microscopie électronique montre la nature composite des pastilles et l'incrustation des cristaux de métronidazole dans la matrice inorganique poreuse au niveau de sites bien différenciés. Les cristaux de métronidazole y sont soit encapsulés, soit placés sur une ligne de porosité continue.

L'utilisation du naphtalène comme matériau porogène a conduit à l'obtention de macropores ovoïdes dont la dimension est associée au choix effectué sur la taille des grains de naphtalène, compris entre environ 100 et environ 160 μm. Par ailleurs, il a déjà été signalé que la présence de naphtalène lors du compactage confère un caractère multidirectionnel à l'orientation des grains d'aragonite permettant d'éviter la fracture transversale des pastilles. Des essais effectués en l'absence de composé porogène mettent en évidence de telles fractures.

Le mode de réalisation de l'invention ici décrit n'a pas pour objet de réduire la portée de celle-ci. Il pourra donc être envisagé d'y apporter des modifications sans sortir de son cadre tel que défini par les revendications annexées. Notamment, il pourra être envisagé d'utiliser une autre substance médicamenteuse autre qu'un antibiotique tel que le métronidazole, à partir du moment où cette substance pourra être incorporée avant ou après la fabrication dudit composite poreux. Il pourra bien sûr également être envisagé d'utiliser un autre agent porogène que du naphtalène, quoique celui-ci reste l'agent porogène préféré.

**Revendications**

1. Composite utilisable en tant que matériau de reconstitution osseuse **caractérisé en ce qu'**il est constitué par l'association d'aragonite synthétique présentant des pores et d'au moins une substance médicamenteuse.

2. Composite selon la revendication 1 **caractérisé en ce que** l'aragonite synthétique représente entre 85 % et 99,5 % de la masse totale dudit composite.

3. Composite selon l'une des revendications 1 ou 2 **caractérisé en ce que** ladite substance médicamenteuse est un antibiotique.

4. Composite selon la revendication 3 **caractérisé en ce que** ledit antibiotique est du métronidazole.

5. Procédé d'élaboration d'un composite selon l'une quelconque des revendications 1 à 4 **caractérisé en ce qu'**il consiste à réaliser un mélange comprenant des grains d'aragonite synthétique et au moins un agent porogène, à compacter ledit mélange et à chauffer le produit obtenu de façon à éliminer ledit agent porogène, au moins une substance médicamenteuse étant incluse avant ou après ladite étape d'élimination dudit agent porogène.

6. Procédé selon la revendication 5 **caractérisé en ce que** ledit agent porogène est un composé solide se présentant sous forme de poudre.

7. Procédé selon la revendication 6 **caractérisé en ce que** ladite poudre est constituée de grains présentant un diamètre moyen compris entre 100 µm et 1000 µm.

8. Procédé selon l'une des revendications 6 ou 7 **caractérisé en ce que** ledit agent porogène est du naphtalène.

9. Procédé selon l'une des revendications 5 à 8 **caractérisé en ce que** ladite étape de chauffage est effectuée à des températures de l'ordre de 110°C lorsque ladite substance médicamenteuse est introduite avant ladite étape d'élimination dudit agent porogène.

10. Procédé selon l'une des revendications 5 à 8 **caractérisé en ce que** ladite étape de chauffage est effectuée à des températures de l'ordre de 400°C ou plus lorsque ladite substance médicamenteuse est introduite après ladite étape d'élimination dudit agent porogène.

11. Procédé selon l'une des revendication 5 à 10 **caractérisé en ce que** ladite étape de compactage est effectuée par pressage uniaxial, sous environ 900 MPa (9 kbar).

12. Procédé de synthèse d'aragonite synthétique pour la réalisation d'un composite selon l'une des revendications 1 à 4 **caractérisé en ce qu'**il consiste à mettre en contact de l'hydrogénocarbonate de potassium avec du chlorure de calcium à raison de 2 moles d'hydrogénocarbontate de potassium pour une mole de chlorure de calcium, à l'ébullition et à pH compris entre 7 et 9.

**Patentansprüche**

1. Zusammensetzung, die als Material zur Wiederherstellung von Knochensubstanz eingesetzt werden kann, **dadurch gekennzeichnet, dass** sie aus synthetischem Aragonit, der Poren aufweist sowie aus mindestens einem medizinisch aktiven Stoff besteht.

2. Zusammensetzung nach Anspruch 1, **dadurch gekennzeichnet, dass** der synthetische Aragonit zwischen 85 % und 99,5 % der Gesamtmasse dieser Zusammensetzung liegt.

**3.** Zusammensetzung nach einem der Ansprüche 1 oder 2,
**dadurch gekennzeichnet, dass** es sich beim medizinisch aktiven Stoff um ein. Antibiotikum handelt.

**4.** Zusammensetzung nach Anspruch 3,
**dadurch gekennzeichnet, dass** es sich beim Antibiotikum um Metronidazol handelt

**5.** Verfahren zur Herstellung einer Zusammensetzung nach einem der Ansprüche 1 bis 4,
**dadurch gekennzeichnet, dass** sie in der Verwirklichung eines Gemisches besteht, welches synthetische Aragonitkörner und mindestens ein Mittel zur Porenerzeugung umfasst, ferner im Verdichten dieses Gemisches und im Erwärmen des gewonnenen Produktes, um das Mittel zur Porenerzeugung zu eliminieren, wobei mindestens ein medizinisch aktiver Stoff vor oder nach dem Schritt zum Eliminieren des Mittels zur Porenerzeugung zugegeben wird.

**6.** Verfahren nach Anspruch 5,
**dadurch gekennzeichnet, dass** das Mittel zur Porenerzeugung eine feste Zusammensetzung ist, die als Pulver vorliegt.

**7.** Verfahren nach Anspruch 6,
**dadurch gekennzeichnet, dass** das Pulver aus Kömem mit einem durchschnittlichen Durchmesser zwischen 100 _m und 1000 _m gebildet wird.

**8.** Verfahren nach einem der Ansprüche 6 oder 7,
**dadurch gekennzeichnet, dass** es sich beim Mittel zur Porenerzeugung um Naphthalin handelt.

**9.** Verfahren nach einem der Ansprüche 5 bis 8,
**dadurch. gekennzeichnet, dass** der Erwärmungsschritt bei Temperaturen in der Größenordnung von etwa 110 °C erfolgt, wenn der medizinisch aktive Stoff vor der Eliminierung des Mittels zur Porenerzeugung zugegeben wird.

**10.** Verfahren nach einem der Ansprüche 5 bis 8,
**dadurch gekennzeichnet, dass** der Erwärmungsschritt bei Temperaturen in der Größenordnung von etwa 400 °C oder mehr erfolgt, wenn der medizinisch aktive Stoff nach der Eliminierung des Mittels zur Porenerzeugung zugegeben wird.

**11.** Verfahren nach einem der Ansprüche 5 bis 10,
**dadurch gekennzeichnet, dass** der Verdichtungsschritt durch Pressen in Richtung einer Achse bei einem Druck von etwa 900 MPa (9 kbar) erfolgt

**12.** Verfahren zum Synthetisieren von Kunstaragonit zum Herstellen einer Zusammensetzung nach einem der Ansprüche 1 bis 4,
**dadurch gekennzeichnet, dass** es darin besteht, Kaliumhydrogenkarbonat mit Kalziumchlorid zusammenzubringen, im Verhältnis von 2 Mol Kaliumhydrogenkarbonat für einen Mol Kalziumchlorid, bei Siedetemperatur und bei einem pH-Wert zwischen 7 und 9.

**Claims**

**1.** Composite that can be used as a bone reconstruction material, **characterised in that** it is made up of a combination of synthetic aragonite having pores and at least one medicinal substance.

**2.** Composite according to claim 1 **characterised in that** the synthetic aragonite represents between 85 % and 99.5 % of the total mass of the said composite.

**3.** Composite according to one of claims 1 or 2 **characterised in that** the said medicinal substance is an antibiotic.

**4.** Composite according to claim 3 **characterised in that** the said antibiotic is metronidazole.

5. Method of producing a composite according to any one of Claims 1 to 4 **characterised in that** it consists of producing a mixture comprising grains of synthetic aragonite and at least one porogenic agent, compacting the said mixture and heating the product obtained so that the said porogenic agent is eliminated, at least one medicinal substance being included before or after the step of eliminating the said porogenic agent.

6. Method according to Claim 5 **characterised in that** the said porogenic agent is a solid compound in the form of a powder.

7. Method according to Claim 6 **characterised in that** the said powder is made up of particles having a mean diameter of between 100 µm and 1000 µm.

8. Method according to one of Claims 6 or 7 **characterised in that** the said porogenic agent is naphthalene.

9. Method according to one of Claims 5 to 8 **characterised in that** said heating step is carried out at temperatures of the order of 110°C when the said medicinal substance is introduced before the said step of eliminating the said porogenic agent.

10. Method according to one of Claims 5 to 8 **characterised in that** said heating step is carried out at temperatures of the order of 400°C or more when the said medicinal substance is introduced after the said step of eliminating the said porogenic agent.

11. Method according to one of Claims 5 to 10 **characterised in that** the said compacting step is carried out by uniaxial pressing at about 900 MPa (9 kbar).

12. Method for synthesising synthetic aragonite for the production of a composite according to one of Claims 1 to 4 **characterised in that** it consists of bringing potassium hydrogencarbonate into contact with calcium chloride at the rate of 2 moles of potassium hydrogencarbonate to one mole of calcium chloride at boiling point and at a pH of between 7 and 9.

Fig. 1

Fig. 2

**Fig. 3**

**Fig. 4**